# EUROPEAN PATENT SPECIFICATION

(11) **EP 3 041 485 B1**
(45) Date of publication and mention of the grant of the patent: **03.06.2020**
(21) Application number: 14840445.2
(22) Date of filing: 29.08.2014
(51) Int. Cl.: A61K 35/28, A61K 35/44, A61B 17/88, A61L 27/36, A61L 27/38

(54) **CELL-SEEDED COMPOSITIONS AND METHODS USEFUL FOR TREATING BONE REGIONS**
ZELLBESIEDELTE ZUSAMMENSETZUNGEN UND VERFAHREN ZUR BEHANDLUNG VON KNOCHENREGIONEN
COMPOSITIONS ENSEMENCÉES DE CELLULES ET PROCÉDÉS UTILES POUR TRAITER DES RÉGIONS OSSEUSES

(30) Priority: 02.09.2013 US 201361872828 P
(43) Date of publication of application: 13.07.2016
(73) Proprietor: Muffin Incorporated, West Lafayette, IN 47906 (US)
(72) Inventor: WALLACE, Shelley L., Athens, Georgia 30605 (US); TAYLOR, Amanda F., West Lafayette, IN 47906 (US); CHARLEBOIS, Steven, West Lafayette, IN 47906 (US); STEINHART, Christine M., Ramona, California 92065 (US); FEARNOT, Neal E., West Lafayette, IN 47906 (US)
(74) Representative: Miller, David James
(86) International application number: PCT/US2014/053501
(87) International publication number: WO 2015/031809

(56) References cited:
- WO-A1-2011/133959
- WO-A2-2007/099534
- WO-A2-2007/119240
- WO-A2-2012/040408
- US-A1- 2004 258 670
- RACKWITZ, L. ET AL.: 'Stem cell - and growth factor-based regenerative therapies for avascular necrosis of the femoral head' STEM CELL RESEARCH & THERAPY vol. 3, no. 1, 2012, pages 1 - 9, XP055326263
- BUENO, E. M. ET AL.: 'Cell -free and cell -based approaches for bone regeneration' NATURE REVIEWS RHEUMATOLOGY vol. 5, 2009, pages 685 - 697, XP008130692
- PAPATHANASOPOULOS, A. ET AL.: 'Biological considerations of mesenchymal stem cells and endothelial progenitor cells' INJURY vol. 39, no. SUPPL., 2008, pages S21 - S32, XP025562787

## Description

### REFERENCE TO RELATED APPLICATION

This application claims the benefit of U.S. Provisional Patent Application No. 61/872,828, filed September 2, 2013.

### BACKGROUND

The present disclosure relates to treating diseased or damaged tissue in a subject, and in particular aspects to compositions for use in treating bone regions in patients, for example for use in the treatment of ischemic and/or necrotic regions of bone as can occur in avascular necrosis.

Avascular necrosis (AVN) is characterized by areas of dead trabecular bone, often involving the subchondral plate. This degradation of bone underlying articular and load-bearing surfaces in the body can cause loss of support for the cartilage, leading to severe deterioration of the joint. Severe joint destruction resulting from osteonecrosis is seen in 50% of the patients, and a major surgical matter10% of the 500,000 total hip replacements performed annually in the United States are intended to treat AVN. AVN most commonly affects the hip joint and can often occur in younger patients before the cartilage is worn enough to require a total or partial joint replacement. After the supporting bone under an articular surface has de- graded, damage to the cartilage often follows, resulting in a painful, unstable joint. Current treatment is primarily limited to debridement and autologous bone grafting, which is technically challenging in which the surgeon harvests cancellous bone from the patient, requiring a second incision and comorbidity. Replacement of the femoral head or total joint arthroplasty is needed if the bone grafting procedure is unsuccessful or contraindicated.

While significant effort has been made to improve treatment in this and other areas related to diseased or damaged bone, progress has been slow. Needs exists for improved and/or alternative compositions and methods useful for the repair of repair of deficient regions of bone, including those resulting from AVN.

WO 2007/119240 A2 describes a cell preparation comprising endothelial progenitor cells and mesenchymal stem cells. The preparation may be combined with a conductive material, e.g. amorphous calcium phosphate or a natural suitable scaffold such as a fibrin matrix. The composition is intended for use in repairing a bone defect by locally applying the cell preparation to an area of bone defect.

### SUMMARY

The scope of this invention is defined by the claims. Embodiments in the description relating to methods of treatment are not covered by the claims. In certain aspects, the present disclosure relates to compositions for use in the treatment of bone tissue. Preferred compositions are intended for use in the treatment of trabecular bone tissue exhibiting avascular necrosis, for example to induce new bone growth in regions of such tissue.

Accordingly, in some of its embodiments, the present disclosure provides a composition for use in treating a region bone of a patient exhibiting avascular necrosis, including implanting in the region endothelial progenitor cells and mesenchymal stem cells, combined with a collagenous extracellular matrix (ECM) tissue material that includes native heparin from a source tissue for the collagenous ECM tissue material. The composition includes endothelial progenitor cells relative to mesenchymal stem cells in a respective ratio of at least 2:1, or at least 3:1. In certain forms, such ratio is in the range of about 2:1 to about 10:1. The collagenous ECM tissue can retain additional native bioactive substances from a source tissue, including glycoproteins, glycosaminoglycans, proteoglycans and growth factors (e.g. FGF-2), in addition to retaining native heparin from the source tissue.

In additional embodiments, the present disclosure concerns compositions including the components as summarized above and/or as identified elsewhere in this disclosure, and methods for preparing such compositions.

Additional embodiments as well as features and advantages thereof will be apparent to those skilled in the pertinent field upon reviewing the disclosures herein.

### DETAILED DESCRIPTION

Reference will now be made to certain embodiments, and specific language will be used to describe the same. It will nevertheless be understood that no limitation of the scope of the invention is thereby intended. Any alterations and further modifications in the described embodiments and any further applications of the principles of the present invention as described herein are contemplated as would normally occur to one skilled in the art to which the invention relates.

As disclosed above, certain aspects of the present invention relate to compositions including a combination of endothelial progenitor cells (EPCs) and mesenchymal stem cells (MSCs), and also an extracellular matrix tissue, methods of preparation thereof, and uses thereof to treat regions of diseased or damaged bone, for example necrotic bone.

As disclosed above, compositions and methods herein will involve the use of endothelial progenitor cells (EPCs). EPCs are immature endothelial cells, which have the capacity to proliferate, migrate, and differentiate into endothelial cells but have not yet acquired characteristics of mature endothelial cells. EPCs include but are not limited to colony forming unit-endothelial cells (CFU-ECs), circulating angiogenic cells (CACs), circulating endothelial precursors (CEPs), and endothelial colony-forming cells (ECFC) including low proliferative potential ECFC (LPP-ECFC) and/or high proliferative ECFC (HPP-ECFC). In preferred aspects, the EPCs are or include ECFCs, such as HPP-ECFCs.

EPCs can be isolated from blood, bone marrow, or cord blood and can be identified in the CD34+ cell fraction in adult human peripheral mononuclear cells. EPCs may be mobilized from bone marrow into peripheral blood (circulating EPCs) in response to certain physiological stimuli, such as, for example, tissue injury. Circulating EPCs can be obtained from adult human blood. In certain aspects, EPCs can be isolated from these or other sources using CD34+ cells or CD133+ cells alone or in combination with KDR+ as an EPC-rich cell fraction in peripheral blood via direct FACS sorting or other available ex-vivo selection method such as magnetic beads, microfluidics, lab-on-a-chip, affinity column or associated device.

As disclosed above, ECFCs are preferred cells for use herein. Desirably, the ECFCs are obtained from umbilical cord blood. ECFCs can be characterized by: expression of the cell surface proteins KDR, CD34, vWF, eNOS, and VE-cadherin, and lack of expression of the hematopoietic cell markers CD45, AC133, CDllb and CD14; the capacity to proliferate at a clonal plating level and replate into secondary and tertiary ECFCs; the ability to incorporate acetylated low density lipoprotein (AcLDL); the capacity to form capillary-like structures in vitro; and/or the capacity to form human blood vessels in vivo in immunodeficient mice and incorporate with murine vasculature to become part of murine systemic circulation. The ECFC cell populations for use herein can be substantially purified ECFCs (prior to combination with the MSCs), for example a clonal ECFC population or a cell population in which at least 90%, or at least 95%, or 100% of the cells exhibit ECFC characteristics as noted above. For additional information about ECFCs and methods for their preparation, reference can be made for example to United States Patent Publication Nos. 20050266556 (December 1, 2005), 20080025956, (January 31, 2008) and 20090280180 (November 12, 2009).

MSCs for use in the invention can be obtained from any suitable source and in any suitable manner. Suitable sources of MSCs include for example placental tissue, bone marrow, dental tissue, testicle tissue, uterine tissue, umbilical cord blood, umbilical cord tissue, and skin tissue. The MSCs can be provided in a mixed population with other cells, for example in an unfractionated bone marrow aspirate or purified bone marrow mononuclear cells. The MSCs can also be provided in a relatively pure population of MSCs, for example wherein at least 90% of the cells in the population express CD105, CD106, CD156, CD44, CD29, CD166, Stro-1, FGF10, Prx1, Oct4, Sox2, and Nanog and do not express CD34, CD45, CD14, and CD31. MSCs obtained from cord blood are preferred. For additional information regarding MSCs and methods for obtaining them, reference can be made for example to United States Patent Publication 20120052049 (March 1,2012).

The EPCs and MSCs can each independently be autologous or allogenic to the patient. Illustratively, in certain embodiments, the EPCs and MSCs are both allogenic to the patient, for example as in the use of allogenic EPCs and allogenic MSCs derived from cord blood. In other embodiments, the EPCs can be autologous to the patient (e.g. obtained from cord blood or peripheral blood of the patient), and the MSCs can be allogenic to the patient (e.g. obtained from allogenic cord blood). In still further embodiments, the EPCs can be allogenic to the patient (e.g. obtained from allogenic cord blood), and the MSCs can be autologous to the patient (e.g. obtained from cord blood, peripheral blood, or bone marrow of the patient such as in the form of bone marrow aspirate containing MSCs, purified bone marrow mononuclear cells containing MSCs, or more purified MSC preparations obtained from bone marrow).

Collagenous extracellular matrix (ECM) material for use herein can be a decellularized animal tissue layer including ECM tissue. In this regard, "decellularized" as used herein refers to a state of the ECM tissue in which all or substantially all of the cells native to the ECM tissue have been removed; thus, other (non-native) cells can be present on or in the ECM tissue, which is nonetheless referred to as decellularized. The ECM tissue layer can be obtained from a source tissue of a warm-blooded vertebrate animal, such as an ovine, bovine or porcine animal. The source tissue layer is preferably a nonmineralized (i.e. soft tissue) source tissue. For example, suitable ECM tissue include those comprising submucosa, renal capsule membrane, dermal collagen, dura mater, pericardium, amnion, fascia lata, serosa, peritoneum or basement membrane layers, including liver basement membrane. Suitable submucosa materials for these purposes include, for instance, intestinal submucosa including small intestinal submucosa, stomach submucosa, urinary bladder submucosa, and uterine submucosa. ECM tissues comprising submucosa (potentially along with other associated tissues) useful in the present invention can be obtained by harvesting such tissue sources and delaminating the submucosa-containing matrix from smooth muscle layers, mucosal layers, and/or other layers occurring in the tissue source. Porcine tissue sources are preferred sources from which to harvest ECM tissues, including submucosa-containing ECM tissues.

ECM tissue used in the invention is preferably decellularized and highly purified, for example, as described in U.S. Patent No. 6,206,931 to Cook et al. or U.S. Patent Application Publication No. US2008286268 dated November 20, 2008, publishing U.S. Patent Application Serial No. 12/178,321 filed July 23, 2008. Preferred ECM tissue material will exhibit an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 5 µg/mg, more preferably less than about 2 µg/mg, and virus levels are preferably less than about 50 plaque forming units (PFU) per gram, more preferably less than about 5 PFU per gram. These and additional properties of submucosa or other ECM tissue taught in U.S. Patent No. 6,206,931 or U.S. Patent Application Publication No. US2008286268 may be characteristic of any ECM tissue used in the present invention.

In certain embodiments, the ECM tissue material used herein will be a membranous tissue with a layer structure as isolated from the tissue source. The ECM tissue can, as isolated, have a layer thickness that ranges from about 50 to about 250 microns when fully hydrated, more typically from about 50 to about 200 microns when fully hydrated, although isolated layers having other thicknesses may also be obtained and used. These layer thicknesses may vary with the type and age of the animal used as the tissue source. As well, these layer thicknesses may vary with the source of the tissue obtained from the animal source.

The ECM tissue material utilized desirably retains a structural microarchitecture from the source tissue, including structural fiber proteins such as collagen and potentially also elastin that can form native fibers. Such fibers can in certain embodiments be non-randomly oriented, as can occur in the source tissue for the decellularized ECM tissue material. Such non-random collagen and/or other structural protein fibers can in certain embodiments provide an ECM tissue that is non-isotropic in regard to tensile strength, thus having a tensile strength in one direction that differs from the tensile strength in at least one other direction.

The decellularized ECM tissue material may include, in addition to native heparin, one or more bioactive agents native to the source of the ECM tissue material and retained in the ECM tissue material through processing. For example, a submucosa or other ECM tissue material may retain one or more native growth factors such as but not limited to basic fibroblast growth factor (FGF-2), transforming growth factor beta (TGF-beta), epidermal growth factor (EGF), cartilage derived growth factor (CDGF), and/or platelet derived growth factor (PDGF). As well, submucosa or other ECM materials when used in the invention may retain other native bioactive agents such as but not limited to proteins, glycoproteins, proteoglycans, and glycosaminoglycans. For example, decellularized ECM tissue materials may include native heparin, heparin sulfate, hyaluronic acid, fibronectin, cytokines, and the like. Decellularized ECM tissue materials retaining native are advantageously used herein, for example because they can exhibit the capacity to bind beneficial growth factors such as those secreted by cells of compositions herein or by cells of the patient at the implant site, for example osteogenic growth factors such as osteogenic bone morphogenic proteins. Thus, generally speaking, a submucosa or other ECM tissue material may retain from the source tissue one or more bioactive components that induce, directly or indirectly, a cellular response such as a change in cell morphology, proliferation, growth, protein or gene expression.

Submucosa-containing ECM materials or other ECM materials used in the present invention can be derived from any suitable organ or other tissue source, usually a soft tissue source (non-bone, non-cartilage) containing connective tissue. The ECM materials processed for use in the invention will typically include abundant collagen, most commonly being constituted at least about 80% by weight collagen on a dry weight basis. Such naturally-derived ECM materials will for the most part include collagen fibers that are non-randomly oriented, for instance occurring as generally uniaxial or multi-axial but regularly oriented fibers. When processed to retain native bioactive factors (e.g. as discussed above), the ECM material can retain these factors interspersed as solids between, upon and/or within the collagen fibers. Particularly desirable naturally-derived ECM materials for use in the invention will include significant amounts of such interspersed, non-collagenous solids that are readily ascertainable under light microscopic examination with appropriate staining. Such non-collagenous solids can constitute a significant percentage of the dry weight of the ECM material in certain inventive embodiments, for example at least about 1%, at least about 3%, and at least about 5% by weight in various embodiments of the invention.

The submucosa-containing or other ECM tissue material used in the present invention may also exhibit an angiogenic character and thus be effective to induce angiogenesis in a host engrafted with the material. In this regard, angiogenesis is the process through which the body makes new blood vessels to generate increased blood supply to tissues. Thus, angiogenic materials, when contacted with host tissues, promote or encourage the formation of new blood vessels into the materials. Methods for measuring in vivo angiogenesis in response to biomaterial implantation have recently been developed. For example, one such method uses a subcutaneous implant model to determine the angiogenic character of a material. See, C. Heeschen et al., Nature Medicine 7 (2001), No. 7, 833-839. When combined with a fluorescence microangiography technique, this model can provide both quantitative and qualitative measures of angiogenesis into biomaterials. C. Johnson et al., Circulation Research 94 (2004), No. 2, 262-268.

Decellularized ECM tissue layers can be used in the invention as single layer implants, but in certain embodiments will be used in multilaminate constructs. In this regard, a variety of techniques for laminating layers together are known and can be used to prepare multilaminate constructs used for the graft in the present invention. For example, a plurality of (i.e. two or more) layers of collagenous material, for example submucosa-containing or other ECM material, can be bonded together to form a multilaminate structure. Illustratively, two to about two hundred decellularized collagenous ECM tissue layers can be bonded together to provide a multilaminate construct for use in the present invention. In certain embodiments, two to eight decellularized collagenous ECM tissue layers are bonded together to form a multilaminate construct for use herein. Preferably submucosa-containing ECM tissue layers are isolated from intestinal tissue, more preferably small intestinal tissue. Porcine-derived tissue is preferred for these purposes. The layers of ECM tissue can be bonded together in any suitable fashion, including dehydrothermal bonding under heated, non-heated or lyophilization conditions, using adhesives, glues or other bonding agents, crosslinking with chemical agents or radiation (including UV radiation), or any combination of these with each other or other suitable methods. For additional information as to multilaminate ECM constructs that can be used in the invention, and methods for their preparation, reference may be made for example to U.S. Patent Nos. 5,711,969, 5,755,791, 5,855,619, 5,955,110, 5,968,096, and to U.S. Patent Publication No. 20050049638 A1 published March 3, 2005. These constructs can be perforated or non-perforated, and when perforated may include an array of perforations extending substantially across the surface of the construct, or may include perforations only in selected areas.

Osteogenic compositions of embodiments herein can incorporate xenograft ECM tissue material (i.e., cross-species material, such as tissue material from a non-human donor to a human recipient), allograft ECM material (i.e., interspecies material, with tissue material from a donor of the same species as the recipient), and/or autograft ECM material (i.e., where the donor and the recipient are the same individual). Further, BMP and/or other exogenous bioactive substances incorporated into an ECM material may be from the same species of animal from which the ECM material was derived (e.g. autologous or allogenic relative to the ECM material) or may be from a different species from the ECM material source (xenogenic relative to the ECM material). In certain embodiments, the ECM tissue material will be xenogenic relative to the patient receiving the graft, and any added cells or other exogenous material(s) will be from the same species (e.g. autologous or allogenic) as the patient receiving the graft. Illustratively, human patients may be treated with xenogenic ECM materials (e.g. porcine-, bovine- or ovine-derived) that have been modified with exogenous human BMP(s) such as rhBMP(s) as described herein.

ECM tissue materials used in embodiments herein can be free or essentially free of additional, non-native crosslinking, or may contain additional crosslinking. Such additional crosslinking may be achieved by photo-crosslinking techniques, by chemical crosslinkers, or by protein crosslinking induced by dehydration or other means. However, because certain crosslinking techniques, certain crosslinking agents, and/or certain degrees of crosslinking can destroy the remodelable properties of a remodelable material, where preservation of remodelable properties is desired, any crosslinking of the remodelable ECM material can be performed to an extent or in a fashion that allows the material to retain at least a portion of its remodelable properties. Chemical crosslinkers that may be used include for example aldehydes such as glutaraldehydes, diimides such as carbodiimides, e.g., 1-ethyl-3-(3-dimethylaminopropyl)carbodiimide hydrochloride, ribose or other sugars, acyl-azide, sulfo-N-hydroxysuccinamide, or polyepoxide compounds, including for example polyglycidyl ethers such as ethyleneglycol diglycidyl ether, available under the trade name DENACOL EX810 from Nagese Chemical Co., Osaka, Japan, and glycerol polyglycerol ether available under the trade name DENACOL EX 313 also from Nagese Chemical Co. Typically, when used, polyglycerol ethers or other polyepoxide compounds will have from 2 to about 10 epoxide groups per molecule.

In additional embodiments, osteogenic compositions herein can incorporate ECM tissue material that has been subjected to a process that expands the tissue material. In certain forms, such expanded materials can be formed by the controlled contact of an ECM material with a denaturing agent such as one or more alkaline substances until the material expands, and the isolation of the expanded material. Illustratively, the contacting can be sufficient to expand the ECM tissue material to at least 120% of (i.e. 1.2 times) its original bulk volume, or in some forms to at least about two times its original volume. Thereafter, the expanded material can optionally be isolated from the alkaline medium, e.g. by neutralization and/or rinsing. The collected, expanded material can be used in any suitable manner in the preparation of a material for administration to a patient. The expanded material can be enriched with bioactive components, comminuted, dried, and/or molded, etc., in the formation of an implantable body of a desired shape or configuration. In certain embodiments, a dried implant body formed with an expanded ECM tissue material can be compressible.

Treatment of an ECM tissue material with a denaturant, such as an alkaline material, can cause changes in the physical structure of the material that in turn cause it to expand. Such changes may include denaturation of the collagen in the material. In certain embodiments, it is preferred to expand the material to at least about three, at least about four, at least about 5, or at least about 6 or even more times its original bulk volume. It will be apparent to one skilled in the art that the magnitude of the expansion is related to several factors, including for instance the concentration or pH of the alkaline medium, the exposure time of the alkaline medium to the material, and temperature used in the treatment of the material to be expanded, among others. These factors can be varied through routine experimentation to achieve a material having the desired level of expansion, given the disclosures herein.

A collagen fibril is comprised of a quarter-staggered array of tropocollagen molecules. The tropocollagen molecules themselves are formed from three polypeptide chains linked together by covalent intramolecular bonds and hydrogen bonds to form a triple helix. Additionally, covalent intermolecular bonds are formed between different tropocollagen molecules within the collagen fibril. Frequently, multiple collagen fibrils assemble with one another to form collagen fibers. It is believed that the addition of an alkaline substance to the material as described herein can be conducted so as to not significantly disrupt the intramolecular and intermolecular bonds, but denature the material to an extent that provides to the material an increased processed thickness, e.g. at least twice the naturally-occurring thickness. ECM materials that can be processed to make expanded materials for use as substrates can include any of those disclosed herein or other suitable ECM's. Typical such ECM materials will include a network of collagen fibrils having naturally-occurring intramolecular cross links and naturally-occurring intermolecular cross links. Upon expansion processing as described herein, the naturally-occurring intramolecular cross links and naturally-occurring intermolecular cross links can be retained in the processed collagenous matrix material sufficiently to maintain the collagenous matrix material as an intact collagenous sheet material; however, collagen fibrils in the collagenous sheet material can be denatured, and the collagenous sheet material can have an alkaline-processed thickness that is greater than the thickness of the starting material, for example at least 120% of the original thickness, or at least twice the original thickness. The expanded ECM material can then be processed to provide foam or sponge substrates for use as or in the graft body, e.g. by comminuting, casting, and drying the processed material. Additional information concerning expanded ECM materials and their preparation is found in United States Patent Application Publication No. US20090326577 published December 31, 2009, publishing United States Patent Application Serial No. 12/489,199 filed June 22, 2009.

In certain embodiments herein, the composition to be administered can consist or consist essentially of the decellularized ECM tissue and a combination of the EPC and MSC cells. Additionally or alternatively, the composition, excluding the cells, can be predominantly comprised of the decellularized ECM tissue, for example at least 80% by weight, at least 90% by weight, or at least 95% by weight, comprised of the decellularized ECM tissue on a dry weight basis.

The ECM tissue material used herein can optionally be in particulate form, for example as incorporated into flowable compositions for administration. Such ECM particulate materials can have particles or random and/or regular shape. Illustratively, random ECM tissue particulates can be prepared by crushing, grinding or chopping a larger decellularized ECM tissue sheet material. On the other hand, a regular ECM tissue particulate can be prepared by controlled cutting of shapes such as circular, ovoid or polygonal shapes from a larger decellularized ECM tissue layer material, e.g. to provide disk form particles. Such regular ECM particles can retain a sheet form, and can in certain embodiments have maximum sheet dimensions (across the face of the sheet particles) in the range of about 0.1 to about 1 mm, or about 0.1 to about 5 mm, or about 0.1 to about 2 mm. In addition or alternatively, the regular ECM particles can be multilaminate constructs containing multiple bonded decellularized ECM layers, for example as can be prepared by controlled cutting, as mentioned above, of corresponding larger multilaminate decellularized ECM tissue constructs. Methods of laminating multiple layers of decellularized ECM layers are described herein and can be used in the generation of the larger multilaminate decellularized ECM tissue constructs to be cut to generate the regular ECM particulate. An ECM particulate can be incorporated with a flowable liquid carrier, typically an aqueous carrier, along with other components herein, to form an injectable or otherwise flowable composition for administration.

In other forms, in addition to ECM tissue materials, compositions herein can include other organic carrier materials. Illustrative materials include, for example, synthetically-produced substrates comprised or natural or synthetic polymers. Illustrative synthetic polymers are preferably biodegradable synthetic polymers such as polylactic acid, polyglycolic acid or copolymers thereof, polyanhydride, polycaprolactone, polyhydroxy-butyrate valerate, polyhydroxyalkanoate, or another biodegradable polymer or mixture thereof. Preferred implant bodies comprised of these or other materials (e.g. ECM materials as discussed herein) will be porous matrix materials configured to allow cellular invasion and ingrowth into the matrix.

Inorganic scaffolding materials can also be incorporated in the compositions herein. In certain embodiments, the compositions can incorporate one or more mineral-containing materials along with the ECM tissue material and bone morphogenic protein. Such mineral material(s) can serve as scaffolding to support the generation of hard tissue such as bone. Many mineral-containing materials for such purposes are known and can be used, for example in particulate form. Suitable materials include for instance hydroxyapatite, tricalcium phosphate, bioglass, calcium phosphate, calcium sulfate, bone, or combinations thereof.

A mineral-containing material and the ECM tissue material can be combined in any suitable manner. In some variants, the mineral-containing material is a particulate material, such as a powder or granular material, and the ECM tissue material is also a particulate material. In these forms, the mineral-containing particulate and the ECM tissue particulate can be in admixture with one another, preferably in a substantially homogenous admixture. Such admixtures can be provided in dry form for later combination with cell populations or mixtures thereof as disclosed herein.

The composition to be administered contains a greater number of EPCs relative to MSCs, whereby the ratio of EPCs to MSCs is at least 2:1, or at least 3:1. In certain embodiments, the ratio of EPCs to MSCs will be in the range of 2:1 to about 10:1, desirably in the range of 2:1 to about 5:1. ECFCs are preferred when the MSC and EPC cells are used in such ratios. It will be understood that these ratios of EPCs to MSCs may be the ratios initially applied to seed a collagenous ECM tissue and/or other carrier material, and optionally can also be the ratios upon administration to the patient. Those skilled in the field will understand that the ratio at the time of administration may differ from the ratio at seeding, due to differences in the rate of proliferation (if any) of the EPCs and the MSCs during the incubation period (if any) prior to administration.

In addition or alternatively to the above-recited ratios of EPCs to MSCs, the total number of the EPCs and MSCs (i.e. the sum of the two) administered can be in the range of about 10⁵ to about 10¹⁰ cells, more typically about 10⁵ to about 10⁸ cells.

The EPCs and MSCs, which are desirably karyotypically normal, are used in combination with a collagenous extracellular matrix (ECM) tissue material that includes native heparin from a source tissue for the collagenous ECM tissue material as a solid carrier material. The EPCs, MSCs and solid carrier material can be combined prior to administration to the patient, or they can be separately administered to the patient and combined *in situ,* or combinations thereof. When the EPCs and MSCs are combined with the solid carrier material prior to administration, they can be incubated with the solid carrier material to as to proliferate and expand the number of EPCs and/or MSCs; alternatively, the EPCs and/or MSCs can be combined with the solid carrier material and then administered to the patient without substantial expansion (e.g. 5% or less, or 1% or less) of the number of EPCs and/or MSCs. In certain embodiments, the EPCs and MSCs are combined with the solid carrier matrix and incubated in contact therewith for a sufficient period of time to cause EPCs and MSCs (e.g. at least 50% of the total number of EPCs and MSCs present) to attach to the solid carrier matrix, but without substantial expansion of the number of EPCs or MSCs as noted above, and the resulting composition can be administered to the patient. Decellularized ECM tissue materials are preferred solid carrier matrix materials for these purposes, particularly those in particulate form and retaining native bioactive substances as disclosed hereinabove. In this regard, if other solid carrier matrix materials are used in conjunction with decellularized tissue ECM materials (for example mineral-containing materials as noted above), in certain modes of practice these other solid carrier matrix materials can be present when the EPCs and/or MSCs are incubated with the ECM material, or can be combined with the ECM material (and cells) after the incubation period.

In addition to EPCs, MSCs, and one or more solid carrier materials as discussed above, compositions to be administered can contain other inert or bioactive materials. These include, for example, other cells, organic polymers, bone morphogenic proteins such as BMP-2 or BMP-7, osteonectin, bone sialoproteins (Bsp), alpha.2HS-glycoproteins, bone Gla-protein (Bgp), matrix Gla-protein, bone phosphoglycoprotein, bone phosphoprotein, bone proteoglycan, protolipids, growth factors such as platelet derived growth factor, skeletal growth factor, fibroblast growth factor and the like; particulate extenders; inorganic water soluble salts, e.g. NaCl, calcium sulfate; sugars, e.g. sucrose, fructose and glucose; pharmaceutically acceptable carriers, drugs (e.g. antibiotics); and the like.

Compositions disclosed herein can be used to treat diseased or damaged bone in a patient. For example, the diseased or damaged bone can occur in any of the bones in an animal, especially a mammal such as a human, including flat bones (e.g., ribs and the frontal and parietal bones of the cranium), long bones (e.g., bones of the extremities), short bones (e.g., wrist and ankles bones), irregular bones (e.g., vertebrae and the pelvis), and sesamoid bones (e.g., the patella). Damaged bone to be treated can include fractured bone. Diseased bone to be treated can in some embodiments include osteopenic bone, osteoporotic bone, necrotic bone, or ischemic bone.

In certain preferred forms herein the compositions described herein will be used to treat a bone region exhibiting avascular necrosis (AVN). AVN may have any of a variety of known causes, such as trauma, systemic disease, adverse drug or radiation response, rheumatoid, or occupational hazard (e.g. prolonged exposure to high pressure). A bone region exhibiting AVN can occur in bone associated with an articulating joint, for example the femoral head of the hip, the femoral condyle, the neck of the talus, or the waist of the scaphoid bone. These and other AVN-exhibiting bone regions are often regions of trabecular bone (also known as cancellous bone) regions.

The compositions described herein can be delivered to the bone region to be treated in any suitable fashion. Delivery by injection through a needle lumen, or otherwise by passage through the lumen of a delivery device such as a tube or catheter, is preferred.

In especially preferred embodiments herein, the composition is delivered into AVN-exhibiting trabecular bone of the femoral head of a hip bone. Desirably, the AVN is at a stage prior to structural collapse of the femoral head. The delivery of the composition can be conducted by injection through a needle such as a trocar or other needle. In certain embodiments, the composition is delivered through a multi-needle array which deploys two, three or more individual needle cannulae in a radially-extending array to provide a regionalized delivery of the composition. In still other embodiments, the composition can be delivered in conjunction with the conduct of a core decompression in which a trough is drilled or otherwise surgically created in the femoral neck. The composition described herein can be used in or as a material to fill the trough to treat the AVN-exhibiting bone region. Alternatively, the trough can be filled with a synthetic or natural bone graft material such as tricalcium phosphate, bone cement, or autologous or allogenic bone, and a composition as described herein can thereafter be delivered through the graft-filled trough and into the AVN-exhibiting bone region, or example by injection through a needle passed through the graft-filled trough. These and other modes of treatment of AVN-exhibiting bone regions will be apparent to those skilled in the field from the descriptions herein.

For the purpose of promoting a further understanding of embodiments herein and features and advantages thereof, the following specific Examples are provided. It will be understood that these Examples are illustrative, and not limiting, of the scope of embodiments otherwise described herein.

### EXAMPLES

Implant preparation and surgery A 4mm diameter disk was cut from an ECM sheet material (a 4-layer laminate of renal capsule, "RC") with a 4-mm biopsy punch. The RC disk implant was incubated at 37°C for 24 hours in contact with a 20µl suspension prepared by combining cord blood-derived ECFCs (about 300,000 cells in 10µl) and cord blood-derived MSCs (about 100,000 cells in 10µl). The cell-seeded RC disk was then passed into the surgical field to allow the surgeon to implant it into a prepared defect of a known immunodeficient (SCID) mouse calvarial defect model. For the model, bilateral 4mm diameter defects were drilled in each mouse. One of the bilateral defects was used as a control (receiving no treatment material) and the other received the treatment material. Two lengths of titanium wire were tied to the outer edge of the implant disc on opposed sides as imageable references. Sutures were routed through cranial and caudal suture holes drilled in the treatment defects and through mated holes in the implant disk. The implant disk was secured to parietal bone with a suture knot. Control (void) treatment sites were similarly tied with sutures but received no implant. The incisions were closed, and the mouse was fitted with an Elizabethan collar (worn for 1 week after surgery). The mice in the study were imaged *in vivo* with microCT (small scale computed tomography) at 2, 4, 8, 12 and 16 weeks post implant. The microCT scans were used the change in bone coverage for the treated and untreated defects.

Results: For defects treated with the cell-seeded ECM implant disks the percent bone coverage at 16 weeks post implant averaged 52.6% (n=6). For the corresponding untreated defects in these groups, the percent bone coverage at 16 weeks post implant averaged 23.7%. Also, in experiments similarly conducted, a group of mice (n=7) receiving just the ECM implant disk (no added cells) on one side and no treatment on the other side averaged about 35% bone coverage on the treated side and about 22% bone coverage on the untreated side at 16 weeks post implant.

As used in this specification and the appended claims, the singular forms "a," "an" and "the" include plural reference unless the context clearly dictates otherwise. Unless defined otherwise all technical and scientific terms used herein have the same meaning as commonly understood to one of ordinary skill in the art to which this invention belongs.

Where a range of values is provided, it is understood that each intervening value, to the tenth of the unit of the lower limit, unless the context clearly dictates otherwise, between the upper and lower limit of that range and any other stated or intervening value in that stated range, is encompassed within the invention. The upper and lower limits of these smaller ranges may independently be included in the smaller ranges, and such embodiments are also encompassed within the invention, subject to any specifically excluded limit in the stated range. Where the stated range includes one or both of the limits, ranges excluding either or both of those included limits are also included in the invention.

## Claims

1. A composition for treating damaged or diseased bone in a patient, comprising endothelial progenitor cells (EPCs) and mesenchymal stem cells (MSCs), wherein the composition includes the EPCs and MSCs in a respective cell number ratio of at least 2:1, wherein the composition also comprises a solid carrier material, and wherein the solid carrier material comprises a collagenous extracellular matrix (ECM) tissue material that includes native heparin from a source tissue for the collagenous ECM tissue material.

2. The composition of claim 1, wherein at least 50% of the total number of EPCs and MSCs in the composition are attached to the solid carrier material.

3. The composition of claim 1, wherein the composition includes the EPCs and MSCs in a respective cell number ratio in the range of 2:1 to 10:1.

4. The composition of any preceding claim, wherein the EPCs are endothelial colony forming cells (ECFCs).

5. The composition of any preceding claim, wherein at least one of the EPCs and the MSCs is allogenic to the patient, preferably wherein the MSCs are allogenic to the patient and the EPCs are allogenic to the patient.

6. The composition of any one of claims 1 to 4, wherein at least one of the EPCs and the MSCs is autologous to the patient.

7. The composition of any preceding claim, wherein the MSCs are cord blood derived MSCs and/or wherein the EPCs are cord blood derived EPCs.

8. The composition of any one of claims 1 to 6, wherein the EPCs are cord blood derived EPCs and wherein the MSCs are bone marrow-derived MSCs.

9. The composition of any preceding claim, wherein the ECM tissue material retains native growth factors, glycosaminoglycans, proteoglycans and glycoproteins from a source tissue for the ECM tissue material.

10. The composition of any preceding claim, wherein the collagenous ECM tissue material:
(i) comprises submucosal tissue or renal capsule tissue and/or
(ii) is a porcine ECM tissue material.

11. The composition of any preceding claim, wherein the composition also comprises a calcium phosphate compound.

12. The composition according to any preceding claim, wherein the MSCs and EPCs are karyotypically normal.

13. The composition of any preceding claim, for use in the treatment of ischemic or necrotic bone.

14. The composition for use according to claim 13, wherein:
(i) the bone exhibits avascular necrosis;
(ii) the bone is in a femoral head of a hip joint; and/or
(iii) the bone is trabecular bone.

15. The composition according to any one of claims 1 to 12 for use in a method of treating a trabecular bone region of a femoral head of a patient, wherein the trabecular bone exhibits avascular necrosis, wherein said method comprises:
passing a delivery device having a lumen into the trabecular bone region; and
delivering said composition through the lumen and into the trabecular bone region.

16. The composition for use according to claim 15, wherein said method further comprises drilling a passageway through the femoral head, preferably wherein said method further comprises filling at least a portion of the passageway with said composition.

## Patentansprüche

1. Zusammensetzung zur Behandlung von beschädigtem oder erkranktem Knochen bei einem Patienten, die endotheliale Vorläuferzellen (EPCs) und mesenchymale Stammzellen (MSCs) umfasst, wobei die Zusammensetzung die EPCs und MSCs in einem jeweiligen Zellzahlverhältnis von wenigstens 2:1 beinhaltet, wobei die Zusammensetzung auch ein festes Trägermaterial umfasst und wobei das feste Trägermaterial ein kollagenes ECM-(extrazelluläres Matrix)-Gewebematerial umfasst, das natives Heparin von einem Ausgangsgewebe für das kollagene ECM-Gewebematerial beinhaltet.

2. Zusammensetzung nach Anspruch 1, wobei wenigstens 50 % der Gesamtzahl der EPCs und MSCs in der Zusammensetzung an das feste Trägermaterial angelagert sind.

3. Zusammensetzung nach Anspruch 1, wobei die Zusammensetzung die EPCs und MSCs in einem jeweiligen Zellzahlverhältnis im Bereich von 2:1 bis 10:1 beinhaltet.

4. Zusammensetzung nach einem vorherigen Anspruch, wobei die EPCs endotheliale koloniebildende Zellen (ECFCs) sind.

5. Zusammensetzung nach einem vorherigen Anspruch, wobei die EPCs und/oder die MSCs für den Patienten allogen sind, wobei vorzugsweise die MSCs für den Patienten allogen sind und die EPCs für den Patienten allogen sind.

6. Zusammensetzung nach einem der Ansprüche 1 bis 4, wobei die EPCs und/oder MSCs für den Patienten autolog sind.

7. Zusammensetzung nach einem vorherigen Anspruch, wobei die MSCs von Nabelschnurblut abgeleitete MSCs sind und/oder wobei die EPCs von Nabelschnurblut abgeleitete EPCs sind.

8. Zusammensetzung nach einem der Ansprüche 1 bis 6, wobei die EPCs von Nabelschnurblut abgeleitete EPCs sind und wobei die MSCs von Knochenmark abgeleitete MSCs sind.

9. Zusammensetzung nach einem vorherigen Anspruch, wobei das ECM-Gewebematerial native Wachstumsfaktoren, Glykosaminoglykane, Proteoglykane und Glykoproteine von einem Ausgangsgewebe für das ECM-Gewebematerial beibehält.

10. Zusammensetzung nach einem vorherigen Anspruch, wobei das kollagene ECM-Gewebematerial:
(i) submuköses Gewebe oder Nierenkapselgewebe umfasst und/oder
(ii) ein porcines ECM-Gewebematerial ist.

11. Zusammensetzung nach einem vorherigen Anspruch, wobei die Zusammensetzung auch eine Calciumphosphatverbindung umfasst.

12. Zusammensetzung nach einem vorherigen Anspruch, wobei die MSCs und EPCs karyotypisch normal sind.

13. Zusammensetzung nach einem vorherigen Anspruch zur Verwendung bei der Behandlung von ischämischem oder nekrotischem Knochen.

14. Zusammensetzung zur Verwendung nach Anspruch 13, wobei:
(i) der Knochen eine avaskuläre Nekrose aufweist;
(ii) der Knochen sich in einem Oberschenkelkopf eines Hüftgelenks befindet; und/oder
(iii) der Knochen trabekulärer Knochen ist.

15. Zusammensetzung nach einem der Ansprüche 1 bis 12 zur Verwendung in einem Verfahren zur Behandlung einer trabekulären Knochenregion eines Oberschenkelkopfes eines Patienten, wobei der trabekuläre Knochen eine avaskuläre Nekrose aufweist, wobei das Verfahren Folgendes beinhaltet:
Einführen einer Zuführungsvorrichtung mit einem Lumen in die trabekuläre Knochenregion; und
Zuführen der genannten Zusammensetzung durch das Lumen in die trabekuläre Knochenregion.

16. Zusammensetzung zur Verwendung nach Anspruch 15, wobei das genannte Verfahren ferner das Bohren eines Durchgangs durch den Oberschenkelkopf beinhaltet, wobei das genannte Verfahren vorzugsweise ferner das Füllen von wenigstens einem Teil des Durchgangs mit der genannten Zusammensetzung beinhaltet.

## Revendications

1. Composition destinée à traiter un os endommagé ou malade chez un patient, comprenant des cellules progénitrices endothéliales (CPE) et des cellules souches mésenchymateuses (CSM), dans laquelle la composition inclut les CPE et CSM dans un rapport de nombres respectifs de cellules d'au moins 2/1, dans laquelle la composition comprend aussi un matériau de support solide, et dans laquelle le matériau de support solide comprend un matériau de tissu de matrice extracellulaire collagène (MEC) qui inclut de l'héparine native d'un tissu source pour le matériau de tissu de MEC collagène.

2. Composition selon la revendication 1, dans laquelle au moins 50 % du nombre total de CPE et de CSM dans la composition sont attachés au matériau de support solide.

3. Composition selon la revendication 1, dans laquelle la composition inclut les CPE et les CSM dans un rapport de nombres respectifs de cellules dans la plage de 2/1 à 10/1.

4. Composition selon l'une quelconque des revendications précédentes, dans laquelle les CPE sont des cellules formant des colonies endothéliales (CFCE).

5. Composition selon l'une quelconque des revendications précédentes, dans laquelle au moins l'une des CPE et CSM est allogénique pour le patient, de préférence les CSM sont allogéniques pour le patient et les CPE sont allogéniques pour le patient.

6. Composition selon l'une quelconque des revendications 1 à 4, dans laquelle au moins l'une des CPE et CSM est autologue pour le patient.

7. Composition selon l'une quelconque des revendications précédentes, dans laquelle les CSM sont des CSM dérivées de sang de cordon et/ou dans laquelle les CPE sont des CPE dérivées de sang de cordon.

8. Composition selon l'une quelconque des revendications 1 à 6, dans laquelle les CPE sont des CPE dérivées de sang de cordon et dans laquelle les CSM sont des CSM dérivées de la moelle osseuse.

9. Composition selon l'une quelconque des revendications précédentes, dans laquelle le matériau de tissu de MEC conserve des facteurs de croissance, des glycosaminoglycanes, des protéoglycanes et des glycoprotéines natifs d'un tissu source pour le matériau de tissu de MEC.

10. Composition selon l'une quelconque des revendications précédentes, dans laquelle le matériau de tissu de MEC collagène :
(i) comprend un tissu sous-muqueux ou un tissu de capsule rénale et/ou
(ii) est un matériau de tissu de MEC porcin.

11. Composition selon l'une quelconque des revendications précédentes, dans laquelle la composition comprend aussi un composé de phosphate de calcium.

12. Composition selon l'une quelconque des revendications précédentes, dans laquelle les CSM et les CPE sont normales d'un point de vue caryotypique.

13. Composition selon l'une quelconque des revendications précédentes, destinée à être utilisée dans le traitement d'un os ischémique ou nécrotique.

14. Composition destinée à être utilisée selon la revendication 13, dans laquelle :
(i) l'os révèle une nécrose avasculaire ;
(ii) l'os est dans une tête fémorale d'une articulation de hanche ; et/ou
(iii) l'os est un os trabéculaire.

15. Composition selon l'une quelconque des revendications 1 à 12 destinée à être utilisée dans un procédé de traitement d'une région d'os trabéculaire d'une tête fémorale d'un patient, dans laquelle l'os trabéculaire révèle une nécrose avasculaire, dans laquelle ledit procédé comprend :
le passage d'un dispositif de délivrance ayant une lumière jusque dans la région d'os trabéculaire ; et
la délivrance de ladite composition à travers la lumière et jusque dans la région d'os trabéculaire.

16. Composition destinée à être utilisée selon la revendication 15, dans laquelle ledit procédé comprend en outre le forage d'une voie de passage à travers la tête fémorale, de préférence dans laquelle ledit procédé comprend en outre le remplissage d'au moins une partie de la voie de passage avec ladite composition.
